# EUROPÄISCHE PATENTSCHRIFT

(11) **EP 0 343 168 B1**
(45) Veröffentlichungstag und Bekanntmachung des Hinweises auf die Patenterteilung: **07.04.1993**
(21) Anmeldenummer: 88900811.6
(22) Anmeldetag: 19.01.1988
(51) Int. Cl.: A61N 1/00

(54) **VORRICHTUNG ZUM ABSAUGEN VON WUNDFLÜSSIGKEITEN**
WOUND FLUID ASPIRATING DEVICE
DISPOSITIF POUR ASPIRER LES FLUIDES DES BLESSURES

(30) Priorität: 20.01.1987 DE 3701386; 02.04.1987 DE 3711093; 24.07.1987 DE 3724483; 30.10.1987 DE 8714456 U
(43) Veröffentlichungstag der Anmeldung: 29.11.1989
(60) Teilanmeldung: 92104631.4
(73) Patentinhaber: MEDINORM AKTIENGESELLSCHAFT MEDIZINTECHNISCHE PRODUKTE, D-66287 Quierschied (DE)
(72) Erfinder: FELL, Helmut, D-6607 St. Ingbert (DE)
(74) Vertreter: Müller, Hans, Dipl.-Ing.
(86) Internationale Anmeldenummer: DE8800022
(87) Internationale Veröffentlichungsnummer: WO8805319

(56) Entgegenhaltungen:
- EP-A- 0 061 723
- EP-A- 0 066 699
- WO-A-00/2182
- DE-B- 1 060 098
- DE-C- 69 607
- FR-A- 2 186 215
- FR-A- 2 208 545
- FR-A- 2 560 770
- US-A- 3 556 044

## Beschreibung

### TECHNISCHES GEBIET

Die Erfindung betrifft eine Vorrichtung zum Absaugen von Wundflüssigkeiten. Derartige Vorrichtungen werden bei der Wunddrainage eingesetzt. Dazu wird eine vorevakuierte Saugflasche, vorzugsweise mit Vakuumanzeige, über einen Verbindungsschlauch mit einem perforierten Wunddrainageschlauch verbunden, der in eine luftdicht verschlossene Wundhöhle eingebracht ist.

### STAND DER TECHNIK

Saugflaschen sind in der Regel fabrikationsseitig auf etwa fünf Prozent bis zehn Prozent des normalen Luftdrucks eingestellt und weisen typischerweise einen Inhalt von 150 ml bis 1000 ml auf. Der Innendruck wird über eine Vakuumanzeige angezeigt. Soll nur schwach gesaugt werden, wird die vom Hersteller relativ stark evakuierte Flasche teilbelüftet bevor sie an den Verbindungsschlauch angeschlossen wird.

Füllt sich die Flasche während des Gebrauchs mit Sekret, steigt der Druck der Luft im Restvolumen nach dem Boyle-Mariotte'schen Gesetz langsam hyperbolisch an. Bei einem Ausgangsdruck der leeren Flasche von fünf Prozent des Atmosphärendruckes herrscht eine Druckdifferenz zwischen innen und außen von 95 Prozent des Atmosphärendruckes. Füllt man die Flasche zur Hälfte, verdoppelt sich ihr Innendruck, so daß die Druckdifferenz 90 Prozent des Atmosphärendruckes beträgt. Bei einer 75 Prozent gefüllten Flasche beträgt die Duckdifferenz zwischen innen und außen immer noch 80 Prozent des Atmosphärendruckes. Diese Rechnung ist eine Näherung, die nicht den Dampfdruck des Sekrets berücksichtigt, der jedoch klein gegenüber dem Luftdruck ist. Von leerer bis zu 75 Prozent mit Sekret gefüllter Flasche saugt das System also recht gleichmäßig, was für die Wirkung wichtig ist. Der Bereich gleichmäßiger Saugwirkung wird jedoch dann erheblich schlechter, wenn die in bezug auf Sekret leere Flasche bereits teilbelüftet ist, um nur schwach zu saugen. Neben diesem Nachteil weist die vom Benutzer teilbelüftete Flasche den Nachteil auf, daß Kontaminationsgefahr besteht. Daher werden teilweise auch vom Hersteller Saugflaschen geliefert, deren Innendruck etwa 50 Prozent des Atmosphärendruckes entspricht.

Die üblicherweise verwendete Anschlußeinrichtung ist ein bloßer Schlauch, der mit seinem einen Ende auf einen Anschlußstutzen auf der Saugflasche und mit seinem anderen Ende an einen Anschlußstutzen am Wunddrainageschlauch angeschlossen wird. Zum Unterbrechen der Anschlußeinrichtung, also des Schlauches, wird eine Klemmeinrichtung verwendet.

Ist ein perforierter Wunddrainageschlauch über einen Verbindungsschlauch mit einer Saugflasche verbunden und wird der Durchgang durch den Verbindungsschlauch durch Aufheben der Wirkung der Klemmeinrichtung hergestellt, steht der volle Unterdruck der vorevakuierten Saugflasche in der Wundhöhle an. Damit insbesondere bei frischen Wunden nicht zu stark gesaugt wird, liefern die Hersteller von Saugflaschen bereits teilbelüftete Flaschen, deren Saugwirkung jedoch schnell nachläßt, wenn sie sich mit Sekret füllen.

Aus der FR-A-2.186.215 ist eine Absaugvorrichtung der eingangs genannten Art bekannt, bei der die verwendete Saugflasche mit einer geringen Menge Wasser gefüllt und dieses Wasser zum Kochen gebracht wird, wobei die in der Flasche befindliche Luft herausgetrieben wird. Anschließend wird die Flasche verschlossen und abgekühlt. Durch Kondensation des Wasserdampfes bildet sich in der Flasche ein Vakuum. Diese Vorrichtung ist für Anwendungsfälle gedacht, wie beispielsweise der Intrauterinabsaugung, bei denen jeweils ein sehr hohes Vakuum erwünscht wird. Eine exakte Voreinstellung des jeweiligen Vakuums ist dabei von untergeordneter Bedeutung.

### DARSTELLUNG DER ERFINDUNG

Ausgehend von diesem Stand der Technik liegt der Erfindung die Aufgabe zugrunde, eine Absaugvorrichtung anzugeben, deren Saugunterdruck auf einfache Weise genau voreingestellt werden kann.

Diese Erfindung ist durch die Merkmale des Patentanspruchs 1 gegeben. Vorteilhafte Weiterbildungen sind Gegenstand von Unteransprüchen.

Die Erfindung geht damit von der Erkenntnis aus, daß durch unterschiedliche Mischungsanteile von Pentan und Hexan sich der jeweils gewünschte Saugunterdruck beliebig voreinstellen läßt. Durch mehr oder weniger starkes Erwärmen der voreingestellten Saugflasche läßt sich darüber hinaus der Saugunterdruck noch nachträglich, beispielsweise im Anwendungsfall, variieren. Unterdruckeinstellungen während des Betreibens einer Saugflasche waren bei herkömmlichen Saugflashen nicht möglich. Ein weiterer Vorteil solcher Ausführungsformen ist darin begründet, daß die Flaschen gegen Verformungen bei erhöhten Temperaturen, z. B. während ihres Sterilisierens, stabiler sind.

Die druckeinstellende Flüssigkeit kann nach einer Weiterbildung der Erfindung auch in einer separaten Ampulle hermetisch verschlossen so bezüglich des Innenraumes der Saugflasche vorhanden sein, daß erst durch Zerbrechen der Ampulle die Flüssigkeit freigesetzt und ihre druckmindernde Wirkung ausgelöst wird.

### KURZE BESCHREIBUNG DER FIGUREN

- Fig. 1:: Perspektivische Darstellung eines Wunddrainagesystems mit einer Saugflasche, in der eine geringe Menge Flüssigkeit mit vorgegebenen geringem Dampfdruck vorhanden ist;
- Fig. 2:: perspektivische Darstellung des oberen Teiles einer Saugflasche entsprechend der wie in Fig. 1, jedoch mit einem zusätzlichen Aufnahmeraum für eine Ampulle mit einer Flüssigkeit mit geringem Dampfdruck;

### WEGE ZUM AUSFÜHREN DER ERFINDUNG

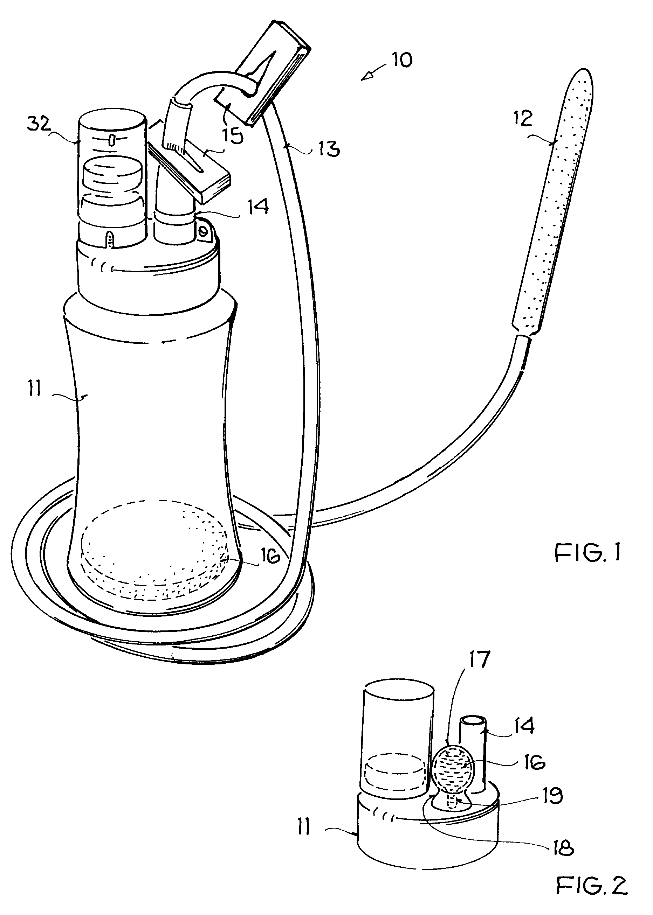

Das in Fig. 1 dargestellte Saugsystem 10 weist eine Saugflasche 11 und einen Wunddrainageschlauch 12 auf, der dadurch mit der Saugflasche 11 verbunden ist, daß ein Verbindungsschlauch 13 auf einen Saugstutzen 14 an der Saugflasche 11 geschoben ist und an sein anderes Ende der Wunddraingeschlauch 12 angeschlossen ist. Das flaschenseitige Ende des Verbindungsschlauches 13 und das Gummiverbindungsstück zur Flasche sind über Schlauchklemmen 15 abquetschbar, was beim Flaschenwechsel ausgenutzt wird.

Im Gegensatz zu herkömmlichen Systemen befindet sich im Innenraum der Saugflasche 11 nicht Luft unter geringem Druck, sondern ein möglichst geringes Volumen einer Flüssigkeit 16, die mit ihrer Gasphase, also ihrem gesättigten Dampf im Gleichgewicht ist. Der Dampf füllt das Restvolumen der Saugflasche 11 aus. Die Flüssigkeit stellt eine Mischung von Pentan und Hexan dar.

Der Dampfdruck einer Flüssigkeit ist bekanntlich bei Vorhandensein der flüssigen Phase nicht vom Volumen des Dampfes abhängig. Die in die Saugflasche 11 einzubringende Flüssigkeit 16 wählt man nach dem gewünschten Saugunterdruck aus. Soll der Sauginnendruck bei Zimmertemperatur z.B. 50 Prozent des normalen Atmosphärendruckes betragen, kann ein Gemisch von 50 Prozent Pentan und 50 Prozent Hexan verwendet werden. Wird der Anteil von Pentan erhöht, wird schächere Saugwirkung erzielt, wird der Anteil von Hexan erhöht, steigt die Saugwirkung, fällt also der Sauginnendruck. Ein Volumen von etwa 1 ml in einer Flasche von 600 ml reicht aus, die gesamte Flasche mit Dampf zu füllen. Eine Menge von etwa 5 ml wird bevorzugt
Der Verlauf des Druckes beim Füllen der Saugflasche 11 mit Wundsekret hängt davon ab, ob die eingebrachte Flüssigkeit 16 im Wundsekret löslich ist oder nicht, und wenn sie löslich ist, von dem Verhältnis der Volumina von Flüssigkeit 16 und Wundsekret. Ist die eingebrachte Flüssigkeit nur wenig oder gar nicht in Wundsekret löslich, bleibt im Verlauf des Füllens der Saugflasche 11 mit Sekret der Sauginnendruck weitgehend konstant, d.h. unabhängig vom Füllvolumen. Das bedeutet, daß das Volumen der Flasche trotz höherem Anfangsdruck ganz genutzt werden kann, im Gegensatz zu den Verhältnissen bei luftgefüllten Flaschen mit erhöhtem Anfangsdruck. Ist die eingebrachte Flüssigkeit 16 im Wundsekret löslich, nimmt der Dampfdruck in der Saugflasche 11 mit zunehmenden Füllgrad ab, und zwar folgt er dem Raoult'schen Gesetz der Dampfdruckerniedrigung, welches besagt, daß die relative Dampfdruckerniedrigung bei konstanter Temperatur gleich dem Verhältnis der Zahl der gelösten Moleküle zur Gesamtzahl der Moleküle ist. Daher sinkt im Falle der Löslichkeit der Sauginnendruck der Flasche 11 mit zunehmender Sekretfüllung ab, d.h. sie saugt stärker. Ein solches Erhöhen der Saugwirkung mit zunehmender Füllung entspricht dem gewünschten Idealverlauf, daß nämlich bei frischer Wunde wenig gesaugt wird, und bei älterer Wunde stärker. Auch in diesem Fall wird das Volumen der Saugflasche 11 gut genutzt.

Ein weiterer Vorteil einer Saugflasche 11 mit Flüssigkeit 16 besteht darin, daß der Sauginnendruck durch Temperaturänderung gesteuert werden kann. Steigert man die Temperatur der Saugflasche 11, im einfachsten Fall, indem man sie nahe dem Körper des Patienten anbringt, wird die Saugwirkung geringer da der Innendruck ansteigt. Kühlt man sie ab, saugt sie stärker.

Auch in Zusammenhang mit Sterilisiervorgängen weist eine Saugflasche mit druckeinstellender Flüssigkeit Vorteile auf. Herkömmliche Saugflaschen müssen verhältnismäßig dickwandig ausgeführt werden, damit das bei Temperaturerhöhung weicher werdende Kunststoffmaterial auch beim Sterilisieren der Druckdifferenz zwischen innen und außen widerstehen kann. Bei der meint angewandten Gassterilisation werden bei bekannten Flaschen meistens etwa 35 Grad Celsius angewandt, wobei jedoch eine höhere Temperatur günstiger wäre, von dem thermoplastischen Kunststoff der Flaschen aber nicht ohne Verformung überstanden werden kann. Bei Saugflaschen mit Flüssigkeit hat die Flasche 11 schon bei Zimmertemperatur einen höheren Innendruck als die bisher verwendeten weitgehend evakuierten Flaschen. Dieser Druck steigt beim Erwärmen während der Gassterilisation weiter an und wird beim Siedepunkt der Flüssigkeit 16 gleich dem Atmosphärendruck, so daß jegliche Druckbelastung der Flasche 11 entfällt.

Daher kann die Flasche 11 geringere Wandstärke aufweisen als bekannte Flaschen und dennoch beim Sterilisieren auf höhere Temperaturen erwärmt werden. Bei der oben erwähnten Mischung von 50 Prozent Pentan und 50 Prozent Hexan liegt der Siedepunkt bei Atmosphärendruck bei etwa 50 Grad Celsius, so daß ohne weiteres Sterilisiertemperaturen möglich sind, die erheblich über den bisher anwendbaren Temperaturen um 35 Grad Celsius liegen.

Als druckeinstellende Flüssigkeiten 16 kommen fast ausschließlich organische Flüssigkeiten in Frage. Bei diesen besteht ganz allgemein das Problem der Verträglichkeit mit dem thermoplastischen Kunststoff der Saugflasche 11. Die meisten organischen Flüssigkeiten greifen solche Kunststoffe an, wenn auch sehr langsam. Auch Kleber, wie sie zum Verbinden verschiedener Teile einer Saugflasche verwendet werden, werden langsam angegriffen. Nachteile, die daraus entstehen könnten, werden durch eine Ausführungsform gemäß Fig. 2 umgangen. Bei dieser Ausführungsform ist an der Oberseite der Saugflasche 11 eine elastische Kappe 17 vorhanden, die über einen Verbindungsstutzen 18 mit dem Innenraum der Saugflasche 11 in Verbindung steht. In der elastischen Kappe 17 befindet sich eine Glasampulle 19, in die eine dampfdruckeinstellende Flüssigkeit 16 in vorgegebener Menge eingeschmolzen ist. Bei unbeschädigter Ampulle 19 arbeitet das System wie eine herkömmliche Saugflasche. Soll die Saugkraft ohne Teilbelüftung und damit ohne Verringerung des absaugbaren Sekretvolumens verringert werden, zerbricht man die Ampulle 19, wodurch die Flüssigkeit 16 freigesetzt wird und die Saugflasche 11 mit ihrem Dampf so lange füllt, bis Gleichgewicht zwischen dem Dampfdruck der Flüssigkeit, und dem Innendruck in der Saugflasche 11 hergestellt ist. Auf diese Weise wird die oben anhand von Fig. 1 dargestellte und beschriebene Situation erhalten.

## Patentansprüche

1. Vorrichtung zum Absaugen von Wundflüssigkeiten mit einer Saugflasche (11), die eine Einrichtung zum Konstanthalten des Unterdruckes in der Flasche im wesentlichen über deren gesamten Füllvorgang enthält,
**dadurch gekennzeichnet,** daß
die Flasche (11) mit einer geringen Menge einer Flüssigkeit (16) aus einer Mischung von Pentan und Hexan, deren Dampfdruck dem gewünschten Saugunterdruck entspricht.

2. Vorrichtung nach Anspruch 1,
**dadurch gekennzeichnet,** daß
- die Flüssigkeit (16) in einer Glasampulle (19) eingeschlossen vorhanden ist,
- die Glasampulle (19) im Inneren der Saugflasche (11) vorhanden ist.

3. Vorrichtung nach Anspruch 2,
**dadurch gekennzeichnet,** daß
- eine elastische Kappe (17) an der Oberseite der Saugflasche (11) vorhanden ist,
- diese Kappe (17) über einen Verbindungsstutzen (18) mit dem Innenraum der Saugflasche (11) verbunden ist,
- in der Kappe (17) die Glasampulle (19) vorhanden ist.

## Claims

1. Apparatus for drawing off wound fluids with a suction flask (11) which contains a device for keeping constant the negative pressure in the flask substantially throughout its entire filling process, characterised in that the flask (11) [lacuna] with a small amount of a fluid (16) of a mixture of pentane and hexane whose vapour pressure corresponds to the desired negative suction pressure.

2. Apparatus according to Claim 1, characterised in that the fluid (16) is present in a manner enclosed in a glass ampoule (19), and in that the glass ampoule (19) is present in the interior of the suction flask (11).

3. Apparatus according to Claim 2, characterised in that an elastic cap (17) is present on the upper side of the suction flask (11), in that said cap (17) is connected via a connecting nozzle (18) to the interior of the suction flask (11), and in that the glass ampoule (19) is present in the cap (17).

## Revendications

1. Installation pour aspirer des liquides de plaies à l'aide d'une bouteille aspirante (11), qui contient une installation pour maintenir constante le vide partiel qui règne dans la bouteille sensiblement pendant la totalité de son processus de remplissage, caractérisée en ce que la bouteille (11) est pourvue d'une faible proportion d'un liquide (16) constitué d'un mélange de pentane et d'hexane, dont la tension de vapeur correspond au vide partiel d'aspiration souhaité.

2. Installation selon la revendication 1, catactérisée en ce que
- le liquide (16) est présent à l'état enfermé dans une ampoule de verre (19),
- l'ampoule de verre (19) est présente à l'intérieur de la bouteille aspirante (11).

3. Installation suivant la revendication 2, caractérisée en ce que
- une coiffe élastique (17) est présente à la surface supérieure de la bouteille aspirante (11),
- la coiffe (17) est en communication par l'intermédiaire d'un raccord de connexion (18) avec l'espace interne de la bouteille aspirante (11),
- l'ampoule de verre (19) est présente dans la coiffe (17).
